# EUROPEAN PATENT SPECIFICATION

(11) **EP 1 893 221 B1**
(45) Date of publication and mention of the grant of the patent: **16.12.2020**
(21) Application number: 06744405.9
(22) Date of filing: 12.06.2006
(51) Int. Cl.: A23L 33/10, A61K 31/702, A23L 33/11, A23L 33/21

(54) **SYNERGISTIC PREBIOTIC COMPOSITIONS**
SYNERGISTISCHE PROBIOTISCHE ZUSAMMENSETZUNGEN
COMPOSITIONS PREBIOTIQUES SYNERGIQUES

(30) Priority: 13.06.2005 HU 0500582
(43) Date of publication of application: 05.03.2008
(73) Proprietor: Vékony, Dr. Ersébet Borbála Jászberényiné, 1095 Budapest (HU); Jászberényi, Márk, 1025 Budapest (HU); Jázsberényi, Áron, 1025 Budapest (HU); Jászberényi, Sára Zsófia, 1095 Budapest (HU); Jászberényi, Veronika Krisztina, 1095 Budapest (HU)
(72) Inventor: JÁSZBERÉNYI Csaba József, 1095 Hungary (HU)
(74) Representative: Szentpéteri, Zsolt
(86) International application number: PCT/HU2006/000051
(87) International publication number: WO 2006/134409

(56) References cited:
- EP-A- 0 648 425
- WO-A-01/78522
- WO-A-02/082929
- DE-A1- 10 109 708
- US-A1- 2005 118 326

## Description

The present invention relates to synergistic prebiotic compositions, obtainable by the process of the invention, in which fructose polymers of GFₙ or Fₘ structures, either containing a glucose (G) end-group and one or more prebiotic components from a group of prebiotics consisting of modified or unmodified starch and suitable partial hydrolysates thereof, partially hydrolysed inulin, natural oligofructoses, fructo-oligosacharides (FOS), lactulose, galactomannan and suitable hydrolysates thereof, indigestible polydextrose, indigestible dextrin and partial hydrolysates thereof, trans-galacto-oligosaccharides (GOS), xylo-oligosaccharides (XOS), acemannan, lentinan or beta-glucan and partial hydrolysates thereof, polysaccharides P and K (PSP, PSK), tagatose and if desired phytosterols and lecithins are used, optionally with other plant extracts or dried plant powders, mineral components vitamins, amino acids and other addi-Prebiotics are in most cases oligo- and/or polysaccharides that are not digested in the stomach and small intestine and reach the colon more or less intact (Roberfroid MB. Prebiotics: preferential substrates for specific germs? American Journal of Clinical Nutrition, 2001:73(2) 406S-409S). However, colonic bacteria are able to use these compounds (Macfarlane GT, Gibson GR. Metabolic activities of the normal colonic flora. In: Gibson SAW, ed. Human health - the contribution of microorganisms. London: Springer-Verlag, 1994:17-52). The advantageous prebiotic components are capable to increase the amount of probiotic microorganisms in the colonic microflora (Collins MD, Gibson GR. Probiotics, prebiotics, and synbiotics: approaches for modulating the microbial ecology of the gut, American Journal of Clinical Nutrition, 1999:69(5), 1052S-1057S). Together with probiotics-related or independent effects said compositions exert a complex physiological influence in the host (Fedorak RN, Madsen KL. Probiotics and prebiotics in gastrointestinal disorders. Current Opinion in Gastroenterology, 2004:20(2):146-155). These include the positive change in lipid and cholesterol levels, limiting the occurrence and amount of dangerous or disadvantagous resident bacteria (these include the dangerous *Helicobacter pylori* or *clostridia*) or invading colonic bacteria (Alm L. The effect of Lactobacillus acidophilus administration upon survival of Salmonella in randomly selected human carriers. Prog Food Nutr Sci 1983:7:13-7; Gibson GR, Wang X. Regulatory effects of bifidobacteria on other colonic bacteria. J Appl Bacteriol 1994;77:412-20). The said compositions can thereby reduce the risk of the leading causes of death, (Functional Foods, G. R. Gibson, C. M. Williams, eds., 389. pp., Woodhead Publishing Ltd, Abington Hall, Abington, Cambridge, England, 2000/2002) heart and circulatory diseases and colorectal (Reddy BS, Hamid R, Rao CV. Effect of dietary oligofructose and inulin on colonic preneoplastic aberrant crypt foci inhibition. 1997: Carcinogenesis 18:1371-1374) or other cancer (Van Loo J, Clune Y, Bennett M, Collins JK. The SYNCAN project: goals, set-up, first results and settings of the human dietary intervention study. 2005:Brit. J. Nutr. 93(S1), 91-98). Interestingly, the anticancer properties of prebiotics are not limited to colonic events (Taper HS, Roberfroid M. Influence of inulin and oligofructose on breast cancer and tumor growth. J. Nutr. 1999:129:1488S-1491S.) An important part of human nutrition is the consumption of suitable amount of soluble and insoluble fiber. It is known from the literature that it is possible to influence and change the colonic microflora thereby improving the health of the host. The prebiotic-probiotic-synbiotic concept is a clear demostration of these effects (Bengmark S. Pre-, pro- and synbiotics. Current Opinion in Clinical Nutrition and Metabolic Care 2001:4(6):571-579; Bengmark S. Gut microbial ecology in critical illness: is there a role for pre-, pro-, and synbiotics. Current Opinion in Critical Care, 2002: 8: 2).

The gastro-intestinal system of a fetus is sterile. The colonization starts during and after birth and the formation of the complex colonic microflora proceeds for years. In fact, the colonic microflora is a changeable biodynamic ecosystem. By the age of two years, the colon of a child is colonized with a hundred or a couple of hundreds strains of bacteria. This develops further and the colonic microflora then remains a close-knit commensal bacterial ecosystem until the age of about 60 years. After that this systems gets somewhat loose, allowing new (unwanted) members to get attached to the system. This contributes or may contribute to the decline of health of the elderly people,

The microbes in the colon (colonized and planktonic) are capable to exert the following positive effects;
- Suppressing the dangerous colonic microorganisms (invading or colonized) and thereby the production of some of their potentially carcinogenic metabolism products and certain unwanted enzymes).
- Prevention of the colon from attack of dangerous exogenous microorganisms.
- Increase of the ratio of the advantageous probiotic bacteria both in the small intestine and also in the colon.
- Improving the developing colonic microflora of the newborn.
- Prevention of the outbreak and reducing the severity of the symptoms of diarrhoea caused by rotaviruses and other viruses and bacteria.
- Reducing the symptoms of chronic intestinal inflammations (Crohn-disease, IBS, colitis).
- Production of short-chain fatty acids (SCF).
   Production of lactic acid.
- Influence on bile-acid transport and biochemical transformations thereof.
- Partial inhibition of hepatic cholesterol biosynthesis.
- Modulation of insulin production and activity.
- Providing extra food for the colonocytes.
- Regulating the production of immunoglobulins and secretory IgA.
- Suppression of the formation of aberrant crypt foci and further steps of colorectal carcinogenesis.
- Suppression of cancer-causing potential of external chemical carcinogenes.
- Suppression of clostridial formation of potentially carcinogenic secondary bile acids.
- Suppression of allergies (including atopic dermatitis).
- Reducing the symptoms of lactose intolerance and other food allergies.
- Reducing the pH of the colon, thereby making the conditions less suitable for certain pathogens.
- Increasing bowel motility.
- Increasing calcium uptake.

These processes, through various biochemical and physiological pathways, exert a generally advantageous physiological action for the host (in this case the human body). It is noteworthy, however, that said positive physiological effects can also be useful for animals. Therefore, the compositions can also be utilized in fodder and feed additives (Abe F, Ishibashi N, Shimamura S. Effect of administration of bifidobacteria and lactic acid bacteria to newborn calves and piglets. J, Dairy Sci. 1995:78:2838-2846.). In addition to the bioactivity discussed above, said compositions are capable to reduce symptoms of allergy (Noverr MC, Huffnagle GB. Does the microbiota regulate immune responses outside the gut? Trends in Microbiology 2004:12:562-568). Said compositions are able to positively modulate the immune system (Gut Flora, Nutrition and Immunity, Fuller R., Perdigón G., Eds., Blackwell Publishing, 2003).

It is well documented in the literature that phytosterols as well as their hydrogenated counteparts, phytostanols and their esters are capable to reduce the total cholesterol and low density lipoprotein cholesterol (LDL) levels in the human blood. Consumption of plant sterols, however, is not a new phenomenon. In fact, it precedes that of the human development. Our close relative primates continue to consume a much higher amount of phytosterols daily than the modem man. The typically 100 to 300 mg/day plant sterol consumption of humans today is much less than the amount required to achieve a meaningful change in blood lipid parameters.

The effect is complex. It is achieved by the phytosterol inhibition of the absorption of the exogenous (food) cholesterol and also by the inhibition of the reabsorption of the cholesterol transported by the endogenous enterohepatic circulation. These result in increased cholesterol clearance. The cholesterol and phytosterol transport can be modulated by the application of plant sterols in lecithin micelles (Ostlund RE, Jr., Spilburg CA, Stenson WF. Sitostanol administered in lecithin micelles potentially reduces cholesterol absolution in humans, American Journal of Clinical Nutrition, 1999;70:826-831). Phytosterols are also capable to prevent the development of benign prostatic hyperplasia (BPH) (Wilt TJ, MacDonald R, Ishani A. Beta-sitosterol for the treatment of benign prostatic hyperplasia: a systematic review. BJU Int. 1999;83:976-983). The successful and efficient application of phytosterol esters in bakery products for the redaction of plasma LDL-cholesterol has also been documented (Quilez J, Rafecas M, Brufau G, Garcia-Lorda P, Megias I, Bulló M, Ruiz JA, Salas-Salvado J, J. Nutr. 2003:133:3103-3109 and references there cited).

WO 02/082929 A (Raisio Benecol Oy; Wester, Ingmar; Palmu, Tapio; Hopia, Anu; Alho-Leht; 24 October 2002) discloses a synergistic prebiotic composition comprising inulin, beta-glucan and a plant sterol in the form of its fatty acid ester and oat bran besides lecithin and probiotic cultures for lowering cholesterol, said composition being a nutraceutical or pharmaceutical composition in the form of e.g. a food or a beverage or as an additive in food products as well as methods to produce those compositions and products.

US 2005/118326 A1 (Anfinsen Jon R et al; 2 June 2005) discloses a composition comprising inulin and beta-glucan, said composition being a foodstuff or food additive composition as well as methods to produce those compositions and products.

EP-A-0 648 425 (Kraft Foods, Inc; Gen Foods Inc; 19 April 1995) discloses a composition comprising inulin (from chicory), modified and natural starch, as well as including of plant sterols from soybean and/or corn oil (containing omega-3-fatty acids) and further plant extracts (guar gum) as well as emulgators (lecithin) and optionally vitamins, minerals and additives, said composition being a foodstuff, as well as methods to produce those compositions and products.

WO 01/78522 A (The Procter & Gamble Company; 25 October 2001) discloses a composition being a foodstuff for the healthy replacement of unhealthy foodstuffs concerning diseases related to beneficiary effects by influencing the colonal micro flora and mentions several possible ingredients, hinting towards compositions comprising inulin, further prebiotic compounds (e.g. beta-glucan, gums, fructooligosaccharides), including a plant sterol containing vegetable fats and oils (corn, soybean) and fats having a high content in omega-3-fatty acids, further plant extracts as well as emulgators (lecithin, relevant passages see search report), as well as methods to produce those compositions and products.

We have found that a proper combination of prebiotics may exert a synergistic effect. The compositions obtainable by the process according to the present invention may be used as medicaments, cosmetics, food and fodder additives, dietary supplements, as well as prebiotic and symbiotic food and fodder.

The present invention relates to synergistic prebiotic compositions, obtainable by the process of the invention, comprising prebiotic components selected from fructose polymers GFₙ and Fₘ, either containing a glucose (G) end-group, or without this glucose end-group and one or more component of a group of prebiotics consisting of modified or unmodified starch and partial hydrolysates thereof, partially hydrolysed inulin, natural oligofructoses, fructo-oligosaccharides (FOS), lactulose, galactomannan and suitable partial hydrolysates thereof, indigestible polydextrose, acemannan, various gums, indigestible dextrin and partial hydrolysates thereof, trans-galacto-oligosaccharides (GOS), xylo-oligosaccharides (XOS), beta-glucan and partial hydrolysates thereof, together if desired with phytosterol/phytostanol components and their suitable esters, and if desired other plant extracts, mineral components, vitamins and additives.

Preferably, the compositions according to the present invention comprise prebiotic components selected from fructose polymers GFₙ and Fₘ, either containing a glucose (G) end-group, or without this glucose end-group and one or more component of a group of prebiotics consisting of modified or unmodified starch and partial hydrolysates thereof, partially hydrolysed inulin, natural oligofructoses, fructo-oligosaccharides (FOS), lactulose, galactomannan and suitable partial hydrolysates thereof, indigestible polydextrose, acemannan, various gums, indigestible dextrin and partial hydrolysates thereof, trans-galacto-oligosaccharides (GOS), xylo-oligosaccharides (XOS), beta-glucan and partial hydrolysates thereof, together if desired with phytosterol/phytostanol components and their suitable esters, and if desired other plant extracts, mineral components, vitamins and additives.

The fructose polymers of GFₙ or Fₘ structures (G= glucose; F= fructose; n>2; m>2) are linear fructose polymers having either a glucose (G) and-group, or being without this glucose and-group. Oligofructoses are consisted of 3 to 10 carbohydrate units. Above that, chicory inulin contains 10 to 60 carbohydrate units, typically with 27 carbohydrates (fructoses with our without one glucose end-group and a fructose chain). Other plants may produce different fructans. These fructans are capable to increase the number of colonized and planktonic bacteria in the large intestine, This results in a change that those bacteria that are less advantageous or may turn dangerous are suppressed by the higher probiotic colony of bacteria. Depending on the chain length of these fructans or other prebiotics, they can be fermented by probiotic bacteria at different-positions in the colon. We have found that the longer inulins are capable to rich the distal colon and sigmoid colon and exert their anticancer actions in the positions where typically most of the cancerous problems occur. The occurrence of these cancers can be the result of various types of carcinogenesis. It has been demonstrated in the literature that directly induced chemical carcinogenesis can be greatly reduced by probiotic bacteria. The prebiotic compositions of our invention can corroborate this effect by considerably increasing the number of Bifidocateria and other beneficial probiotic strains. The local chemical carcinogenesis can also be the result of the formation of secondary bile acids. These secondary bile acids are often formed upon the action of enzymes produced by resident Clostridia. By probiotic suppression of the number of these bacteria by the compositions according to the invention, the chance of secondary bile acid formation can also be reduced. This can be demonstrated by measuring the faecal primary/secondary bile acid ratio.

Other prebiotics can be selected from a group of prebiotics consisting of various gums (guar gum, xanthan gum, locust been gum), carob seed flour, oat bran, rice bran, barley, modified or unmodified starch and suitable partial hydrolysates thereof, partially hydrolysed inulin, natural or synthetic/biosynthetic oligofructoses, fructo-oligosacharides (FOS), lactulose, galactomannan and suitable hydrolysates thereof, indigestible polydextrose, indigestible dextrin and partial hydrolysates thereof, trans-galacto-oligosaccharides (GOS), xylo-oligosaccharides (XOS), acemannan, lentinan or beta-glucan and partial hydrolysates thereof, polysaccharides P and K (PSP, PSK), tagatose, various fungal oligosaccharides and polysaccharides, together with other components.

Further characteristic of the invention is the application of various phytosterols in these compositions. These phytosterols can he found in various plants. Typically the mixture of phytosterols used in foods are of soy or tall oil origin. Corn fiber oil is also very rich in phytosterols and their derivatives. It is well documented in the scientific literature that a considerable reduction (10-20%) of low density lipoprotein (LDL) and total cholesterol (TC) can be achieved by suitable administration of these phytosterol mixtures, their reduced (phytostanol) counterparts and the corresponding phytosterol and phytostanol esters. It has also been demonstrated that the sterols and stanols on the one hand and their esterified counterparts on the other are all suitable for this purpose. The question remains whether these compounds are soluble in the media of applications.

A further characteristic of the invention is the formation of supramolecular compositions. In the process of the invention, a spontaneous multicomponent supramolecular self- assembly (SMSA) takes place between the components (Jean-Marie Lehn, Perspectives in Supramolecular chemistry: From molecular recognition towards self-organisation, Pure and Applied Chemistry 1994:66:1961-1966). To corroborate this self-assembly, we add lecithins and/or an edible oil or a mixture of edible oils, preferably with omega- 3-fatty acid content during the preparation of these compositions. This results in improved solubility profile of the composition compared to the starting components. We have observed that the synergic effect of our compositions is achieved not only by the joint application of various prebiotics. In certain cases the synergy can be the result of the use of prebiotics, probiotics, phytosterols and derivatives, various plant extracts and powders, edible plant oils and their diglyceride and monoglyceride counterparts, lecithins, amino acids and minerals in supramolecular structures. These supramolecular structures positively influence their stability and transport properties. This supramolecular arrangement represents new qualities of the original components and further corroborates their useful bioactivities by modified solubility, transport and stability in these supramolecular assemblies.

Further embodiments of the present invention are the cases when these compositions incorporate further components belonging to the group of vitamins. Due to the special process, these vitamins can be both water soluble and water insoluble. This allows us to employ vitamins that can exert their own action and they can also corroborate the actions of the aforementioned prebiotic components and also those of the phytosterols. A very important contribution of these vitamin mixtures can be that of their antioxidant properties.

A further possibility is the incorporation of physiologically important elements and trace elements that include but are not limited to calcium, magnesium, zinc, phosphorus, selenium, boron, chromium, copper, potassium, iodine, indium and other useful trace elements.

In a further embodiment of the present invention various extracts and plant powders are incorporated into our compositions, depending on the desired properties according to the end use of said compositions. These compositions according to the present invention can be characterized in that in addition to the discussed prebiotics and phytosterols and lecithins the said further plant extracts or powders are one or more of those of *Panax ginseng* (red, Korean ginseng), *Panax ginseng* (white, Chinese ginseng), *Rhodiola rosea* (golden root), *Panax quinquefolium* (American ginseng), *Eleutherococcus senticosus* (Siberian ginseng), *Cynara scolymus* (artichoke), *Uncaria tomentosa* (Cat's claw), *Lepidium meyenii* (maca, Peruvian ginseng), *Paullinia cupana* (guarana), *Croton lechleri* (Sangre de Grado), *Whitania somnifera* (ashwagandha, Indian ginseng), *Panax japonicus* (Japanese ginseng), *Panax vietnamensis* (Vietnamese ginseng), *Panax trifolius, Panax pseudoginseng, Panax notoginseng, Malpighia glabra* (acerola), *Ylex paraguayiensis* (Yerba mate), *Astragalus membranaceus* (astragalus), *Stevia rebaudiana* (stevia), *Pfaffia paniculata* (Brazilian ginseng, suma), *Ginkgo biloba, Tabebuia impetiginosa* (Pan d'arco), *Echinacea purpurea, Peumus boldus* (boldo), *Gynostemma pentaphyllum* (Jiaogulan, also known as Southern Ginseng or Xiancao), *Sutherlandia frutescens* (African ginseng), *Aloe vera* (aloe), *Cistanche salsa, Cistanche deserticola* (and other *Cistanche* sp.), *Codonopsis pilosula* ("poor man's ginseng."), *Nopal opuntia* (Prickly pear cactus), *Citrus sinensis (Citrus aurantium)* and other members of the citrus family (lemon, lime, tangerine, grapefruit), *Camelia sinensis* (tea), *Plantago psyllium* (psyllium), *Amaranth edulis* and other amaranth sp. (amaranth), *Commiphora mukul* (guggul lipid), *Serenoa repens, Serenoa serrulata* (saw palmetto), *Cordyceps sinensis* (Cordycaps), *Lentinula edodes* (Shitake), *Ganoderma lucidium* (Reishi), *Grifola frondosa* (maitake), *Tremella fuciformis* (Silver ear), *Poria cocos* (Hoelen), *Hericium erinaceus* (Lion's Mane), *Agaricus blazei* (Sun mushroom), *Phellinus linteus* (Mulberry yellow polypore), *Trametes versicolor, Coriolus versicolor* (Turkey tails), *Schizophyllum commune* (Split gill), *Inonotus obliquus* (Cinder conk), Oat bran, rice bran, linseed, garlic, *Ceratonia siliqua* (locust been gum or flour from the seeds of carob tree), *Cyanopsis tetragonoloba* (guar gum, EU Food additive code E412), *Xanthomonas campestris* (xanthan gum). These plant extracts and plant powders are capable to potentiate the bioactivity of these compositions based on prebiotics, phytosterols, lecithins, vitamins and minerals. In given cases it also adds other prebiotics to the aforementioned prebiotic mixtures. These can result in more pronounced bioactivities as prebiotics and also in the chosen other bioactivity directions.

A further characteristic of the process of the present invention is the applied ester scrambling method for the edible oil/phytosterol or edible oil/phytosterol/lecithin systems. In this reaction the heat treatment (with catalysis) allows the scrambling and exchange of ester groups between the triglycerides, the lecithins and the originally unesterified or esterified phytosterols. This corroborates the bioavailability and transport of the components involved.

A further embodiment of our present invention is the use of probiotics to obtain synbiotic compositions wherein to the compositions described earlier one or more probiotic strains of bacteria are added. This allows the formation of synbiotic compositions containing both prebiotic and probiotic elements. These compositions allow the selective food support of the already colonized intestinal bacteria as well as novel probiotics for colonizing mainly the large intestine (colon) and also supplying beneficial planktonic bacteria for the gastrointestinal system. The probiotic bacteria can be omitted or employed depending on the desired end-use of the compositions,
A further possibility is the application of the method of the invention for the formation of compositions by spontaneous multicomponent supramolecular self- assembly for the preparation of cosmetics. The oils are employed individually or in a mixture of the following oils or butters: ostrich oil, evening primrose oil, jojoba oil, macadamia nut oil, shea butter, avocado oil, grapeseed oil, tamanu oil, rose hips oil, pomegranate oil, papaya seed oil, moringa oil, mango butter, argan oil, blackcurrant oil, almond oil, apricot kernel oil, borage oil, coconut oil, hazelnut oil, hemp seed oil, neem oil, olive oil, peach kernel oil, sesame oil, wheatgerm oil.

The compositions according to the invention are prepared in several forms that include beverages as well as solid medicaments, dietary supplements, food additives and foods, as well as cosmetics in various gel forms.

A further embodiment of present invention is pharmaceutical compositions. These pharmaceutical compositions can be prepared in the form of tablets, controlled release tablets, chewing tablets, enteric coated tablets, mucoadhesive vaginal tablets, capsules, gels, sols, solutions, tinctures, sprays, plasters, depending on the proposed application. In a typical embodiment of pharmaceutical compositions tablets are pressed, In these tablet form pharmaceutical preparations the active components are formulated together with diluents, excipients or carriers and disintegrants, selected from calcium carbonate, silicium dioxide, magnesium stearate, and fillers (lactose and dibasic calcium phosphate), and buffers (sodium bicarbonate, calcium carbonate, and sodium citrate), low substituted hydroxypropylcellulose, sodium carboxymethyl cellulose, microcrystalline cellulose, calcium carboxymethyl cellulose and croscarmellose sodium. Preferably, the composition also contains extra-granular components comprising silicon dioxide and a lubricant.

Mucoadhesive vaginal tablets can also be prepared from the basic compositions by directly compressing the natural chitosan, cross-linked with glutaraldehyde and if desired with sodium alginate, together with microcrystalline cellulose, sodium carboxymethylcellulose or the hydrophilic (hydroxypropyl methylcellulose [HPMC]).

Dietary supplements can also be prepared according to our invention comprising the prebiotic compositions with the usual nutritionally acceptable additives.

Further embodiment of our invention are prebiotic or synbiotic beverages that contain our prebiotic compositions together with natural fruit juices or other fluids, including dairy or non-dairy products with the usual nutritionally acceptable additives (sweeteners, acidulants, aromas, colorants).

A further embodiment of our invention are food items including prebiotic, probiotic or synbiotic hamburger, cheese-burger, pizza or other fast food. Due to the thermal stability of our compositions, any of these can be applied to the preparation of the hamburger buns and/or the hamburger meat. The probiotic component can be applied in the cheese or in the dressing. A typical burger according to our invention may contain 50% to 100% of the suggested daily dose of prebiotics, phytosterols, probiotics, certain vitamins and minerals. A prebiotic burger with a probiotic cheese or a probiotic dressing is a symbiotic food item (health food).

The following examples are given as illustrations only of the said invention and in no way should be construed as limiting the subject matter of the present invention.

### Example 1

A phytosterol mixture (soy origin) (10 g) is heated with corn oil (20 g) for 2 hours at 100 °C. Depending on the components a solid acid or other catalyst can be used, Then the mixture is cooled to 20 °C and added upon stirring into a mixture of lecithin (20g, soy origin), water (20 ml) and L-lysine (10 g). Finally, this mixture is further mixed with 100 g prebiotic carbohydrates (80 g inulin, 10 g galacto-oligosaccharide, 8 g fructo-oligosaccharide and 2 g lactulose). Depending on the anticipated end-use, further components can be added that may include plant extracts and plant powders, vitamins, minerals, antioxidants and the usual fillers, stabilizers, adhesion modifiers.

### Example 2

The method is followed described in Example 1 but corn germ oil is used.

### Example 3

The method is followed described in Example 1 but corn fiber oil is used.

### Example 4

This method is followed described in Example 1 but coconut oil is used.

### Example 5

The method is followed described in Example 1 but pumpkinseed oil is used.

### Example 6

The method is followed described in Example 1 but fish oil is used.

### Example 7

The method is followed described in Example 1 but other edible oils or their mixtures are used.

### Example 8

The method is followed described in Examples 1 to 7 but sunflower seed lecithin is used.

### Example 9

The method is followed described in Examples 1 to 7 but egg lecithin is used.

### Example 10

The method is followed described in Examples 1 to 9 but a tall oil phytosterol mixture is used.

### Example 11

The method is followed described in examples 1 to 10 but the prebiotic mixture is 80 g of prebiotic carbohydrates (60 g inulin, 10 g beta-glucan, 8 g *Aloe vera* gel powder and 2 g tagatose).

### Example 12

### Food additive (baking mix)

To any of the basic compositions described in Examples 1-11, salt (NaCl) is added (50 g), followed by ascorbic acid (200 mg), a multivitamin mixture (1 g) and dry instant yeast (20 g) and the mixture thus obtained is thoroughly homogenized.

### Example 13

### Probiotic bakery product (bread)

To the baking flour or flour mixture (700 g) a baking mix, described in Example 12 is added (125 g), followed by water (0.3 to 0.4 liter) and the mixture thus obtained is kneaded into a dough. The amount of water depends on the flour or flour mix used. The dough is then processed and baked in an oven.

### Example 14

### Synbiotic product (pastry)

In this product the dough is the prebiotic and the filling, applied after baking is the probiotic component. The dough is made by the use of any of the compositions described in Examples 1 to 11.

In atypical application, flour (380 g), composition according to Example 1 (120 g), dry yeast (20 g), sugar (25 g), lemon peel (grated, 30 g), eggs (2), margarine (50 g), milk (200 ml) is used to make a dough. This dough is levened, fried in 12 pieces in hot oil and filled (after cooling) with a cream containing the probiotic bacteria.

### Example 15

### Dry feed additive

Any of the compositions described in Examples 1 to 11 is mixed with milled cereals (1 to 5 kg) and to this mixture oily seed industrial byproducts are added to obtain a mixture of 10 kg. This premix can be used in various fodder and dry feed mixtures.

### Example 16

Any of the compositions described in Examples 1 to 11 is mixed with a proprietary composition of extracts of the following herbs and plants (20 g):
*Panax ginseng* (red, Korean ginseng), *Panax ginseng* (white, Chinese ginseng), *Rhodiola rosea* (golden root), *Panax quinquefolium* (American ginseng), *Eleutherococcus senticosus* (Siberian ginseng), *Cynara scolymus* (artichoke), *Uncaria tomentosa* (Cat's claw), *Lepidium meyenii* (maca, Peruvian ginseng), *Paullinia cupana* (guarana), *Croton lechleri* (Sangre de Grado), *Whitania somnifera* (ashwagandha, Indian ginseng), *Astragalus membranaceus* (astragalus), *Pfaffia paniculata* (Brazilian ginseng, suma), *Ginkgo biloba, Tabebuia impetiginosa* (Pau d'arco), *Echinacea purpurea, Peumus boldus* (boldo), *Gynostemma pentaphyllum* (Jiaogulan, also known as Southern Ginseng or Xiancao), *Sutherlandia frutescens* (African ginseng), *Aloe vera* (aloe), *Cistanche salsa, Cistanche deserticola, Codonopsis pilosula.*

### Example 17

The method is followed described in examples 1 to 11 but the oil employed is individually or in a mixture of the following oils or butters: ostrich oil, evening primrose oil, jojoba oil, macadamia nut oil, shea butter, avocado oil, grapeseed oil, tamanu oil, rose hips oil, pomegranate oil, papaya seed oil, moringa oil, mango butter, argan oil, blackcurrant oil, almond oil, apricot kernel oil, borage oil, coconut oil, hazelnut oil, hemp seed oil, neem oil, olive oil, peach kernel oil, sesame oil, wheatgerm oil. These compositions can be applied in the typical cosmetic bases in 1 to 90%.

### Example 18

Pharmaceutical preparation containing one of the compositions of Examples 1 to 11, Example 16 and Example 17 in 20 %, sodium carboxymethylcellulose 26%, sodium alginate 22%, microcrystalline cellulose 23%, hydroxypropyl methylcellulose [HPMC] 3% and chitosan 6%.

### Example 19

Cosmetic composition containing one of the prebiotic compositions of Examples 1 to 11, Example 16 and Example 17 with the usual skin-care and hair-care additives. These compositions can be applied in the typical cosmetic bases usually in 1% to 90%. A typical application in moisturizing cream a composition described in Example 1 applied in the following manner: prebiotic composition 10 part, propylene glycol 4.0 part, methyl paraben 0.2 part, water 60.0 part, triethanolamine 2.0 part glyceryl sterate and PEG 6,0 part, stearate/stearic acid 6.0 part, cetyl alcohol 1.0 part, isopropyl myristate 15.0 part, propyl paraben 0.1 part, dimethicone 1.0 part, fragrance, coloring.

### Example 20

Dietary supplement containing one of the prebiotic compositions of Examples 1 to 11, Example 16 and Example 17 with the usual nutritionally acceptable additives. In a typical embodiment of our invention 180 g of the prebiotic composition described in Example 1 was mixed with a proprietary mixture (100 g) of *Cordyceps sinensis* (Cordycaps), *Lentinula edodes* (Shitake), *Ganoderma lucidium* (Reishi), *Grifola frondosa* (maitake), *Tremella fuciformis* (Silver ear), *Poria cocos* (Hoelen), *Hericium erinaceus* (Lion's Mane), *Agaricus blazei* (Sun mushroom), *Phellinus linteus* (Mulberry yellow polypore), *Trametes versicolor, Coriolus versicolor* (Turkey tails), *Schizophyllum commune* (Split gill), *Inonotus obliquus* (Cinder conk), oat bran, rice bran extracts and powders. After thoroughly mixing the composition thus obtained was filled in capsules or in bottles as loose powder.

### Example 21

Beverage containing one of the prebiotic compositions of Examples 1 to 11, Example 16 and Example 17 with natural fruit juices or other fluids, including dairy or non-dairy products with the usual nutritionally acceptable additives. In a typical application the prebiotic composition described in Example 11 (160 g) was mixed with a proprietary mixture (5 g) of *Pfaffia paniculata* (Brazilian ginseng, suma), *Ginkgo biloba, Tabebuia impetiginosa* (Pau d'arco), *Echinacea purpurea, Peumus boldus* (boldo), *Gynostemma pentaphyllum* (Jiaogulan, also known as Southern Ginseng or Xiancao), *Sutherlandia frutescens* (African ginseng), *Aloe vera* (aloe), *Cistanche salsa, Cistanche deserticola.* The components were thoroughly mixed. A portion of this powder mixture (82.5 g) was added to 0.9 liter of orange juice with pulp. The final volume was corrected to 1 liter.

### Example 22

### Prebiotic, probiotic or synbiotic hamburger or other fast food

Any of the compositions described in Examples 1 to 11 can be applied in the preparation of the hamburger bun and/or the hamburger meat. In the preferred embodiment of the invention one hamburger bun or one hamburger contains 8 g of the composition described in Example 1. The probiotic component can be applied in cheese or dressing.

A prebiotic burger with a probiotic dressing is a synbiotic food item (health food).

## Claims

1. A process for the preparation of a synergistic supramolecular prebiotic composition comprising the following steps:
heating 10 g of a phytosterol mixture of soy or tall oil origin with 20 g edible oil selected from the group consisting of corn oil, corn germ oil, corn fiber oil, coconut oil, pumpkinseed oil and fish oil for 2 hours at 100 °C;
cooling said mixture produced in the previous step to 20 °C;
adding to said mixture produced in the previous step, upon stirring, 20 g of a mixture of natural lecithins, the origin of which is selected from the group consisting of soy, sunflower seed and egg, 20 ml water and 10 g L-lysine;
mixing said mixture produced in the previous step with 100g of a mixture of prebiotic carbohydrates, said mixture consisting of 80 g inulin, 10 g galacto-oligosaccharide, 8 g fructo-oligosaccharide and 2 g lactulose; or a 80 g of a mixture of prebiotic carbohydrates, said mixture consisting of 60 g inulin, 10 g beta-glucan, 8 g Aloe vera gel powder and 2 g tagatose; and, optionally,
adding to said mixture produced in the previous step further components selected from the group consisting of plant extracts, plant powders, vitamins, minerals, antioxidants, fillers, stabilizers and adhesion modifiers.

2. A synergistic supramolecular prebiotic composition, obtainable by the process of Claim 1.

3. A composition obtainable by the method of Claim 1, **characterized in that** the said further plant extracts or plant powders are one or more of those of *Panax ginseng,* also known as red, Korean ginseng; *Panax ginseng,* also known as white, Chinese ginseng; *Rhodiola rosea,* also known as golden root; *Panax quinquefolium,* also known as American ginseng; *Eleutherococcus senticosus,* also known as Siberian ginseng; *Cynara scolymus,* also known as artichoke; *Uncaria tomentosa,* also known as Cat's claw; *Lepidium meyenii,* also known as maca or Peruvian ginseng; *Paullinia cupana,* also known as guarana; *Croton lechleri,* also known as Sangre de Grado; *Whitania somnifera,* also known as ashwagandha or Indian ginseng; *Panax japonicus,* also known as Japanese ginseng; *Panax vietnamensis,* also known as Vietnamese ginseng; *Panax trifolius; Panax pseudoginseng; Panax notoginseng; Malpighia glabra,* also known as acerola; *Ylex paraguayiensis,* also known as Yerba mate; *Astragalus membranaceus,* also known as astragalus; *Stevia rebaudiana,* also known as stevia; *Pfaffia paniculata,* also known as Brazilian ginseng or suma; *Ginkgo biloba; Tabebuia impetiginosa,* also known as Pan d'arco; *Echinacea purpurea; Peumus boldus,* also known as boldo; *Gynostemma pentaphyllum,* also known as Jiaogulan, Southern Ginseng or Xiancao; *Sutherlandia frutescens,* also known as African ginseng; *Aloe vera,* also known as aloe; *Cistanche salsa; Cistanche deserticola* and other *Cistanche* sp.; *Codonopsis pilosula,* also known as "poor man's ginseng."; *Nopal opuntia,* also known as Prickly pear cactus; *Citrus sinensis,* also known as *Citrus aurantium;* other members of the citrus family including lemon, lime, tangerine and grapefruit; *Camelia sinensis,* also known as tea; *Plantago psyllium,* also known as psyllium; *Amaranth edulis* and other amaranth sp., also known as amaranth; *Commiphora mukul,* also known as guggul lipid; *Serenoa repens; Serenoa serrulata,* also known as saw palmetto; *Cordyceps sinensis,* also known as Cordycaps; *Lentinula edodes,* also known as Shitake; *Ganoderma lucidium,* also known as Reishi; *Grifola frondosa,* also known as maitake; *Tremella fuciformis*, also known as Silver ear; *Poria cocos,* also known as Hoelen; *Hericium erinaceus,* also known as Lion's Mane; *Agaricus blazei,* also known as Sun mushroom; *Phellinus linteus,* also known as Mulberry yellow polypore; *Trametes versicolor; Coriolus versicolor,* also known as Turkey tails; *Schizophyllum commune,* also known as Split gill; *Inonotus obliquus,* also known as Cinder conk; oat bran; rice bran; linseed; garlic; *Ceratonia siliqua,* also known as locust been gum; flour from the seeds of carob tree; *Cyanopsis tetragonoloba,* also known as guar gum; *Xanthomonas campestris,* also known as xanthan gum.

4. A food product comprising a composition according to any of Claims 2 to 3.

5. A beverage product comprising a composition according to any of Claims 2 to 3.

6. A nutritional or fodder additive preparation comprising a composition according to any of Claims 2 to 3.

7. A pharmaceutical preparation comprising a composition according to any of Claims 2 to 3 and usually applicable additives.

8. The composition according to any of Claims 2 to 3 for use in the prevention or treatment of diseases, which may develop depending on the amount and nature of probiotic bacteria in the colonic microflora.

9. The composition according to any of Claims 2 to 3 for use according to Claim 8, **characterized in that** the said composition is used for the change of lipid, and cholesterol and bile acid metabolism and transport, and blood lipid and cholesterol levels.

10. The composition according to any of Claims 2 to 3 for use in developing and maintaining a healthy colonic microflora.

11. The composition according to any of Claims 2 to 3 for use in the prevention of gastrointestinal damage caused by therapy with oral antibiotics or other oral antibacterial agents.

12. The composition according to any of Claims 2 to 3 for use according to any of Claims 8 to 12 wherein the prebiotic composition further comprises a probiotic thus providing a symbiotic preparation.

13. Use of a food product according to Claim 4 or a beverage product according to Claim 5 as a component of an infant formula.

14. The composition according to any of Claims 2 to 3, for use in relieving or reducing symptoms of allergies.

15. The composition according to any of Claims 2 to 3, for use in enhancing an immune response, which comprises administering a composition in an amount sufficient to enhance a detectable immune response.

16. The composition according to any of Claims 2 to 3, for use in preventing or curing certain types of cancer.

17. The composition according to any of Claims 2 to 3 for use in corroborating the effect of cholesterol lowering drugs.

18. The composition according to any of Claims 2 to 3 for use in corroborating the effect of antibiotics or antibacterial therapy.

19. The composition according to any of Claims 2 to 3 for use as an immune modulant.

## Patentansprüche

1. Verfahren zum Herstellen einer synergistischen, supramolekularen, probiotischen Zusammensetzung, umfassend die folgenden Schritte:
Erhitzen von 10 g einer Phytosterolmischung aus einem Ursprung aus Soja- oder Tallöl mit 20 g Speiseöl, ausgewählt aus der Gruppe bestehend aus Maisöl, Maiskeimöl, Maisfaseröl, Kokosöl, Kürbiskernöl und Fischöl, für zwei Stunden auf 100 °C;
Abkühlen der im vorherigen Schritt hergestellten Mischung auf 20 °C;
Zugeben von 20 g einer Mischung natürlicher Lecithine, deren Ursprung aus der Gruppe ausgewählt ist, die aus Soja, Sonnenblumenkern und Ei besteht, 20 ml Wasser und 10 g L-Lysin, zu der im vorherigen Schritt hergestellten Mischung;
Mischen der im vorherigen Schritt hergestellten Mischung mit 100 g einer Mischung aus probiotischen Kohlenhydraten, wobei die Mischung aus 80 g Inulin, 10 g Galactooligosaccharid, 8 g Fructooligosaccharid und 2 g Lactulose besteht; oder
80 g einer Mischung aus probiotischen Kohlenhydraten, wobei die Mischung aus 60 g Inulin, 10 g Beta-Glucan, 8 g Aloe-Vera-Gelpulver und 2 g Tagatose besteht; und
gegebenenfalls Hinzufügen weiterer Komponenten, ausgewählt aus der Gruppe, die aus Pflanzenextrakten, Pflanzenpulvern, Vitaminen, Mineralien, Antioxidantien, Füllstoffen, Stabilisatoren und Adhäsionsmodifikatoren besteht, zu der im vorherigen Schritt hergestellten Mischung.

2. Synergistische, supramolekulare, probiotische Zusammensetzung, erhältlich durch das Verfahren nach Anspruch 1.

3. Zusammensetzung, erhältlich durch das Verfahren nach Anspruch 1 und **dadurch gekennzeichnet, dass** die weiteren Pflanzenextrakte oder Pflanzenpulver eines oder mehrere sind aus *Panax ginseng,* auch bekannt als roter koreanischer Ginseng;
*Panax ginseng,* auch bekannt als weißer chinesischer Ginseng;
*Rhodiola rosea,* auch bekannt als Rosenwurz;
*Panax quinquefolium,* auch bekannt als amerikanischer Ginseng;
*Eleutherococcus senticosus,* auch bekannt als sibirischer Ginseng;
*Cynara scolymus,* auch bekannt als Artischocke;
*Uncaria tomentosa,* auch bekannt als Katzenkralle;
*Lepidium meyenii,* auch bekannt als Maca oder Peru Ginseng;
*Paullinia cupana,* auch bekannt als Guarana;
*Croton lechleri,* auch bekannt als Drachenblutbaum;
*Whitania Somnifera,* auch bekannt als Ashwagandha oder Schlafbeere;
*Panax japonicus,* auch bekannt als japanischer Ginseng;
*Panax vietnamensis,* auch bekannt als vietnamesischer Ginseng;
*Panax trifolius;*
*Panax pseudoginseng;*
*Panax notoginseng;*
*Malpighia glabra,* auch bekannt als Acerola;
*Ilex paraguayiensis,* auch bekannt als Mate-Strauch;
*Astragalus membranaceus,* auch bekannt als Mongolischer Tragant;
*Stevia rebaudiana,* auch bekannt als Süßkraut;
*Pfaffia paniculata,* auch bekannt als brasilianischer Ginseng oder Suma;
*Ginkgo biloba;*
*Tabebuia impetiginosa;*
*Echinacea purpurea;*
*Peumus boldus,* auch bekannt als Boldo;
*Gynostemma pentaphyllum,* auch bekannt als Jiaogulan oder Unsterblich keitskraut;
*Sutherlandia frutescens,* auch bekannt als Ballonerbse;
*Aloe Vera,* auch bekannt als Aloe;
*Cistanche salsa;*
*Cistanche deserticola* und andere *Cistanche* sp.;
*Codonopsis pilosula,* auch bekannt als "Ginseng des armen Mannes";
*Nopal opuntia,* auch bekannt als Feigenkaktus;
*Citrus sinensis,* auch bekannt als *Citrus aurantium;*
andere Mitglieder der Zitrusfamilie, einschließlich Zitrone, Limette, Mandarine und Grapefruit;
*Camelia sinensis,* auch bekannt als Tee;
*Plantago Flohsamen,* auch bekannt als Flohsamen;
*Amaranth edulis* und andere Amaranth sp., auch bekannt als Amaranth;
*Commiphora mukul,* auch bekannt als Guggul;
*Serenoa repens;*
*Serenoa serrulata,* auch bekannt als Sägepalme;
*Cordyceps sinensis,* auch bekannt als Chinesischer Raupenpilz;
*Lentinula edodes,* auch bekannt als Shiitake;
*Ganoderma lucidium,* auch bekannt als Reishi;
*Grifola frondosa,* auch bekannt als Maitake;
*Tremella fuciformis,* auch bekannt als Silberohr;
*Poria cocos,* auch bekannt als Poria;
*Hericium erinaceus,* auch bekannt als Löwenmähne;
*Agaricus blazei,* auch bekannt als Mandelpilz;
*Phellinus linteus,* auch bekannt als Feuerschwamm;
*Trametes versicolor;*
*Coriolus versicolor,* auch bekannt als Schmetterlingstramete;
*Schizophyllum commune,* auch bekannt als Gemeiner Spaltblättling;
*Inonotus obliquus,* auch bekannt als Schiefer Schillerporling;
Haferkleie;
Reiskleie;
Leinsamen;
Knoblauch;
*Ceratonia siliqua,* auch bekannt als Johannisbrotbaum;
Mehl aus den Samen des Johannisbrotbaums;
*Cyanopsis tetragonoloba,* auch bekannt als Guar;
*Xanthomonas campestris,* auch bekannt als Xanthan.

4. Lebensmittelprodukt, umfassend eine Zusammensetzung nach einem der Ansprüche 2 bis 3.

5. Getränkeprodukt, umfassend eine Zusammensetzung nach einem der Ansprüche 2 bis 3.

6. Nähr- oder Futterzusatzzubereitung, umfassend eine Zusammensetzung nach einem der Ansprüche 2 bis 3.

7. Pharmazeutische Zubereitung, umfassend eine Zusammensetzung nach einem der Ansprüche 2 bis 3 und üblicherweise anwendbare Zusatzstoffe.

8. Zusammensetzung nach einem der Ansprüche 2 bis 3 zur Verwendung bei der Vorbeugung oder Behandlung von Erkrankungen, die sich in Abhängigkeit von der Menge und Art der probiotischen Bakterien in der Dickdarmmikroflora entwickeln können.

9. Zusammensetzung nach einem der Ansprüche 2 bis 3 zur Verwendung nach Anspruch 8, **dadurch gekennzeichnet, dass** die Zusammensetzung zur Änderung des Lipid- und Cholesterin- und Gallensäurestoffwechsels und -transports sowie der Blutlipid- und Cholesterinspiegel verwendet wird.

10. Zusammensetzung nach einem der Ansprüche 2 bis 3 zur Verwendung bei der Entwicklung und Aufrechterhaltung einer gesunden Dickdarmmikroflora.

11. Zusammensetzung nach einem der Ansprüche 2 bis 3 zur Verwendung bei der Vorbeugung von Magen-Darm-Schäden, die durch die Therapie mit oralen Antibiotika oder anderen oralen antibakteriellen Mitteln verursacht werden.

12. Zusammensetzung nach einem der Ansprüche 2 bis 3 zur Verwendung nach einem der Ansprüche 8 bis 12, wobei die probiotische Zusammensetzung ferner ein Probiotikum umfasst, wodurch ein symbiotisches Präparat bereitgestellt wird.

13. Verwendung eines Lebensmittelprodukts nach Anspruch 4 oder eines Getränkeprodukts nach Anspruch 5 als Bestandteil einer Säuglingsnahrung.

14. Zusammensetzung nach einem der Ansprüche 2 bis 3 zur Verwendung bei der Linderung oder Verringerung von Allergiesymptomen.

15. Zusammensetzung nach einem der Ansprüche 2 bis 3 zur Verwendung bei der Verstärkung einer Immunantwort, umfassend das Verabreichen einer Zusammensetzung in einer Menge, die ausreicht, um eine nachweisbare Immunantwort zu verstärken.

16. Zusammensetzung nach einem der Ansprüche 2 bis 3 zur Verwendung bei der Vorbeugung oder Heilung bestimmter Krebsarten.

17. Zusammensetzung nach einem der Ansprüche 2 bis 3 zur Verwendung bei der Bestätigung der Wirkung von cholesterinsenkenden Arzneimitteln.

18. Zusammensetzung nach einem der Ansprüche 2 bis 3 zur Verwendung bei der Unterstützung der Wirkung von Antibiotika oder einer antibakteriellen Therapie.

19. Zusammensetzung nach einem der Ansprüche 2 bis 3 zur Verwendung als Immunmodulans.

## Revendications

1. Procédé de préparation d'une composition prébiotique supramoléculaire synergique comprenant les étapes suivantes :
le chauffage de 10g d'un mélange de phytostérol d'origine huile de soja ou tall avec 20g d'huile comestible choisie dans le groupe constitué par l'huile de maïs, l'huile de germe de maïs, l'huile de fibres de maïs, l'huile de coco, l'huile de graines de citrouille et l'huile de poisson pendant 2 heures à 100 °C ;
le refroidissement dudit mélange produit à l'étape précédente à 20 °C ;
l'ajout audit mélange produit à l'étape précédente, sous agitation, de 20 g d'un mélange de lécithines naturelles, dont l'origine est choisie dans le groupe constitué par le soja, les graines de tournesol et œuf, 20 ml d'eau et 10 g de L-lysine ;
le mélange dudit mélange produit à l'étape précédente avec 100 g d'un mélange d'hydrates de carbone prébiotiques, ledit mélange étant constitué par 80 g d'inuline, 10 g de galacto-oligosaccharide, 8 g de fructo-oligosaccharide et 2 g de lactulose ; ou 80 g d'un mélange d'hydrates de carbone prébiotiques, ledit mélange étant constitué par 60 g d'inuline, 10 g de bêta-glucane, 8 g de poudre de gel d'Aloe vera et 2 g de tagatose ; et, éventuellement,
l'ajout audit mélange produit à l'étape précédente des composants supplémentaires choisis dans le groupe constitué par les extraits végétaux, les poudres végétales, les vitamines, les minéraux, les antioxydants, les charges, les stabilisants et les modificateurs d'adhérence.

2. Composition prébiotique supramoléculaire synergique, pouvant être obtenue par le procédé de la revendication 1.

3. Composition pouvant être obtenue par le procédé de la revendication 1, **caractérisée en ce que** lesdits autres extraits végétaux ou poudres végétales sont un ou plusieurs de ceux de *Panax ginseng,* également connu sous le nom de ginseng coréen rouge ; *Panax ginseng,* également connu sous le nom de ginseng chinois blanc ; *Rhodiola rosea,* également connue sous le nom de racine dorée ; *Panax quinquefolius,* également connu sous le nom de ginseng américain ; *Eleutherococcus senticosus,* également connu sous le nom de ginseng de Sibérie ; *Cynara scolymus,* également connu sous le nom d'artichaut ; *Uncaria tomentosa,* également connue sous le nom de Liane du Pérou ; *Lepidium meyenii,* également connu sous le nom de maca ou ginseng péruvien ; *Paullinia cupana,* également connue sous le nom de guarana ; *Croton lechleri,* également connu sous le nom de Sang-Dragon ; *Whitania somnifera,* également connue sous le nom d'ashwagandha ou de ginseng indien ; *Panax japonicus,* également connu sous le nom de ginseng japonais ; *Panax vietnamensis,* également connu sous le nom de ginseng vietnamien ; *Panax trifolius ; Panax pseudoginseng ; Panax notoginseng ; Malpighia glabra,* également connue sous le nom d'acérola ; *Ilex paraguayiensis,* également connu sous le nom de yerba mate ; *Astragalus membranaceus,* également connu sous le nom d'astragale ; *Stevia rebaudiana,* également connue sous le nom de stévia ; *Pfaffia paniculata,* également connue sous le nom de ginseng brésilien ou suma ; *Ginkgo biloba* ; *Tabebuia impetiginosa,* également connue sous le nom de Pau d'arco ; *Echinacea purpurea* ; *Peumus boldus,* également connu sous le nom de boldo ; *Gynostemma pentaphyllum,* également connue sous le nom de Jiaogulan, ginseng du sud ou Xiancao ; *Sutherlandia frutescens,* également connu sous le nom de ginseng africain ; *Aloe vera,* également connu sous le nom d'aloès ; *Cistanche* salsa ; *Cistanche deserticola* et d'autres espèces *Cistanche ; Codonopsis pilosula,* également connu sous le nom de « ginseng du pauvre » ; *Nopal opuntia,* également connu sous le nom de figue de barbarie ; *Citrus sinensis,* également connu sous le nom de *Citrus aurantium* ; d'autres membres de la famille des agrumes, comprenant le citron, le citron vert, la mandarine et le pamplemousse ; *Camelia sinensis,* également connue sous le nom de thé ; *Psyllium Plantago,* également connu sous le nom de psyllium ; *Amaranthus edulis* et autres espèces d'amarante, également connues sous le nom d'amarante ; *Commiphora mukul,* également connue sous le nom de lipide guggul ; *Serenoa repens ; Serenoa serrulata,* également connue sous le nom de palmier nain ; *Cordyceps sinensis,* également connu sous le nom de Cordyceps ; *Lentinula edodes,* également connue sous le nom de shiitaké ; *Ganoderma lucidium,* également connue sous le nom de Reishi ; *Grifola frondosa,* également connue sous le nom de maitake ; *Tremella fuciformis,* également connue sous le nom d'oreille d'argent ; *Poria cocos,* également connue sous le nom de Hoelen ; *Hericium erinaceus,* également connu sous le nom de crinière-de-lion ; *Agaricus blazei,* également connu sous le nom de champignon du soleil ; *Phellinus linteus,* également connu sous le nom de polypore jaune du mûrier ; *Trametes versicolor; Coriolus versicolor,* également connu sous le nom de queue de dinde ; *Schizophyllum commune,* également connu sous le nom de lames fendues ; *Inonotus obliquus,* également connu sous le nom de polypore oblique ; le son d'avoine ; le son de riz ; la graine de lin ; l'ail ; *Ceratonia siliqua,* également connue sous le nom de gomme de caroube ; la farine de graines de caroubier ; *Cyanopsis tetragonoloba,* également connu sous le nom de gomme de guar ; *Xanthomonas campestris,* également connu sous le nom de gomme xanthane.

4. Produit alimentaire comprenant une composition selon l'une quelconque des revendications 2 à 3.

5. Produit de boisson comprenant une composition selon l'une quelconque des revendications 2 à 3.

6. Préparation d'additifs nutritionnels ou fourragers comprenant une composition selon l'une quelconque des revendications 2 à 3.

7. Préparation pharmaceutique comprenant une composition selon l'une quelconque des revendications 2 à 3 et des additifs habituellement applicables.

8. Composition selon l'une quelconque des revendications 2 à 3 destinée à être utilisée pour la prévention ou le traitement de maladies, qui peuvent se développer en fonction de la quantité et de la nature des bactéries probiotiques dans la microflore colique.

9. Composition selon l'une quelconque des revendications 2 à 3 destinée à être utilisée selon la revendication 8, **caractérisée en ce que** ladite composition est utilisée pour le changement du métabolisme et du transport des lipides, du cholestérol et des acides biliaires, et des taux sanguins de lipides et de cholestérol.

10. Composition selon l'une quelconque des revendications 2 à 3 destinée à être utilisée pour le développement et le maintien d'une microflore colique saine.

11. Composition selon l'une quelconque des revendications 2 à 3 destinée à être utilisée pour la prévention des dommages gastrointestinaux provoqués par une thérapie à l'aide des antibiotiques oraux ou d'autres agents antibactériens oraux.

12. Composition selon l'une quelconque des revendications 2 à 3 destinée à être utilisée selon l'une quelconque des revendications 8 à 12, la composition prébiotique comprenant en outre un probiotique fournissant ainsi une préparation symbiotique.

13. Utilisation d'un produit alimentaire selon la revendication 4 ou d'un produit de boisson selon la revendication 5 comme composant d'une préparation pour nourrissons.

14. Composition selon l'une quelconque des revendications 2 à 3, destinée à être utilisée pour soulager ou réduire les symptômes d'allergies.

15. Composition selon l'une quelconque des revendications 2 à 3, destinée à être utilisée pour renforcer une réponse immunitaire, qui comprend l'administration d'une composition en une quantité suffisante pour renforcer une réponse immunitaire détectable.

16. Composition selon l'une quelconque des revendications 2 à 3, destinée à être utilisée pour la prévention ou le traitement de certains types de cancer.

17. Composition selon l'une quelconque des revendications 2 à 3 destinée à être utilisée pour corroborer l'effet de médicaments diminuant le cholestérol.

18. Composition selon l'une quelconque des revendications 2 à 3 destinée à être utilisée pour corroborer l'effet d'antibiotiques ou de thérapie antibactérienne.

19. Composition selon l'une quelconque des revendications 2 à 3 destinée à être utilisée comme modulant immunitaire.
